# EUROPEAN PATENT SPECIFICATION

(11) **EP 0 286 525 B1**
(45) Date of publication and mention of the grant of the patent: **27.07.1994**
(21) Application number: 88400800.4
(22) Date of filing: 01.04.1988
(51) Int. Cl.: A61B 19/00, A61M 5/14

(54) **Sterile cassette**
Sterile Kassette
Cassette stérile

(30) Priority: 03.04.1987 US 34787; 07.12.1987 US 129527
(43) Date of publication of application: 12.10.1988
(73) Proprietor: Lokken, Oddvin, New York New York 10016 (US)
(72) Inventor: Lokken, Oddvin, New York, N.Y. 10016 (US)
(74) Representative: Bloch, Gérard

(56) References cited:
- EP-A- 0 082 596
- FR-A- 2 382 900
- GB-A- 2 046 095
- GB-A- 2 140 695
- US-A- 3 782 377
- US-A- 3 850 172
- US-A- 3 900 026
- US-A- 4 250 882
- US-A- 4 275 719
- US-A- 4 299 219
- US-A- 4 399 816
- US-A- 4 500 788

## Description

The present invention relates to an apparatus for maintaining a sterilized operating field and a sterilized cassette therefor.

The need to maintain an operating field free from infectious organisms manifests itself in many ways. Surgical incisions may become infected from airborne organisms or from wound exudates. Trauma sites, e.g. injuries, burns etc., may become infected in the same way. This is particularly true in the case of long term treatment of a patient by means of introducing nutrients, medication or the like via a catheter or cannula.

In addition, there are many instances where a catheter is inserted in a blood vessel during diagnosis or treatment of a patient. Catheters are used to supply fluids intravenously, such as nutrients, pharmaceuticals, dyes etc. Catheters are also used in arteries, such as in angioplasty. In all cases where a needle enters a blood vessel, there is the potential for great harm due to infection. This risk of infection also exists for intramuscular and intralymphatic intubations.

In medical practice today, it is all but impossible to avoid accidental introduction of infectious organisms into a dermal puncture site. When this occurs, the results are serious and sometimes fatal. This can occur in several ways.

First, the patient may harbour infectious organisms on the skin, which can be introduced into the body when the catheter is inserted. Second, airborne organisms can land on the skin after the catether or cannula is in place and can be transported into the blood vessel by movement of the catether or cannula caused by movement of the patient. Third, organisms can be brought to the puncture site by the patient or medical personnel. Introduction of infectious organisms can also occur during removal of the catether or cannula.

In EP-A-0 082 596, a cassette assembly is disclosed, which can be made sterile and comprises
A. a first sterile packaging enclosing
   a sterile cassette comprising an elongated flexible support (5) having top and bottom surfaces, an outer edge (5a) defining the boundary of said support, an inner edge (5b) defining an opening (6) in said support, and means (5c) for attaching said bottom surface to the skin of a patient, and
   elongated wall means (4) projecting from said top surface of said support (5) and extending around said inner edge (5b), said wall means (4) having an inner surface (4c) enclosing said opening (6), said wall means (4) further having an open top (4a), an open bottom (4b), and an entrance (7) at one end.
B. a sterile transparent cover means (20) packaged within a second sterile packaging.

This cassette assembly provides a sealing port for a catheter which is inserted under the patient's skin.

However, since the port provides a sealing in cooperation with the catheter, a user cannot rotate the catheter and, for keeping the efficiency of the sealing, he may not translate it any more, once it has been inserted under the skin. Moreover, this cassette must be viewed as only providing a static post-operation protection after introduction of the catheter under the skin, since, as above mentioned, the catheter cannot be rotated for reaching a precise insertion area of the skin, within the area delimited by the cassette.

The present invention solves this problem by providing a package comprising a sterile cassette containing a needle assembly that is used to puncture the skin and a tubing for connection to the needle assembly after it is inserted. The cassette has an open top and bottom for enclosing the operating field on the patient. After the cassette is installed on the patient, the operating field is irradiated and the open top is closed by a sterile cover. Thereafter, the needle assembly is inserted and connected to the tubing without contaminating the irradiated operating field. To enable the user to manipulate the needle assembly and tubing after the cassette is closed, a flexible pouch or bag is provided and the cover is made transparent. Moreover, a sterilizing gas may be flowed into the cassette as discussed above.

The present invention is illustrated in terms of its preferred embodiments in the accompanying drawings, in which:
Fig. 1 is a view in perspective of the cassette of the present invention with parts broken away for clarity;
Fig. 2 is a plan view of the cassette of Fig. 1 with the top and bottom covers removed;
Fig. 3 is a plan view, of the cassette of Fig. 1 with the cannula inserted into the skin and attached to the tubing, the cover shown being folded back only for clarity;
Figs. 4 and 5 are detail views of the tubing and needle assembly;
Fig. 6 is a detail view in section of the needle assembly support;
Fig. 7 is a detail view of an alternative embodiment of the invention;
Fig. 8 is a detail view of another alternative embodiment of the invention;
Fig. 9 is a view in perspective of another cassette of the present invention with parts broken away for clarity;
Fig. 10A is a detail view in section taken along lines 10A-10A in Fig. 9;
Fig. 10B is a view similar to Fig. 10A, but with the protective sheets of Fig. 9 removed and the cover in place;
Fig. 11 is a plan view of the cassette of Fig. 9 with the cover being shown folded back only for clarity; and
Fig. 12 is a schematic diagram showing the system for flowing sterilizing gas into the cassette.

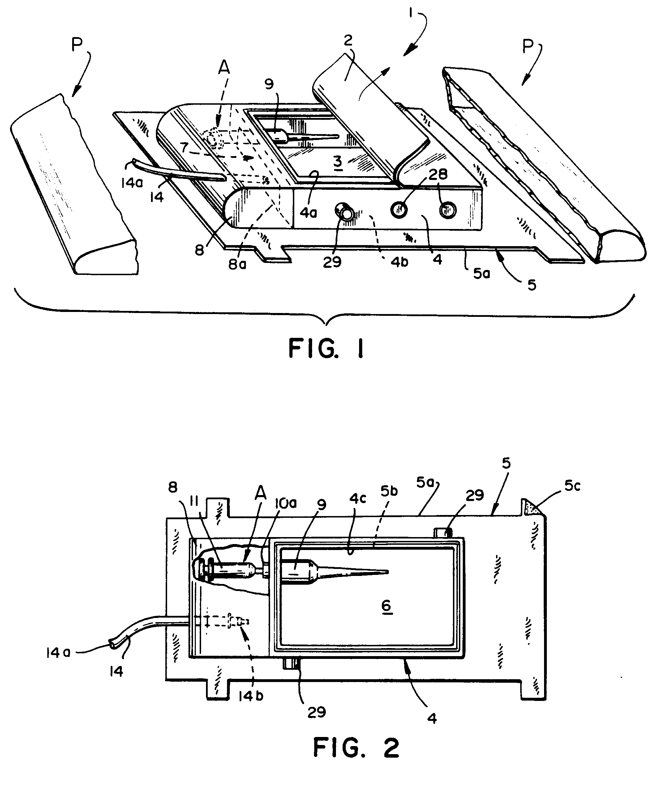

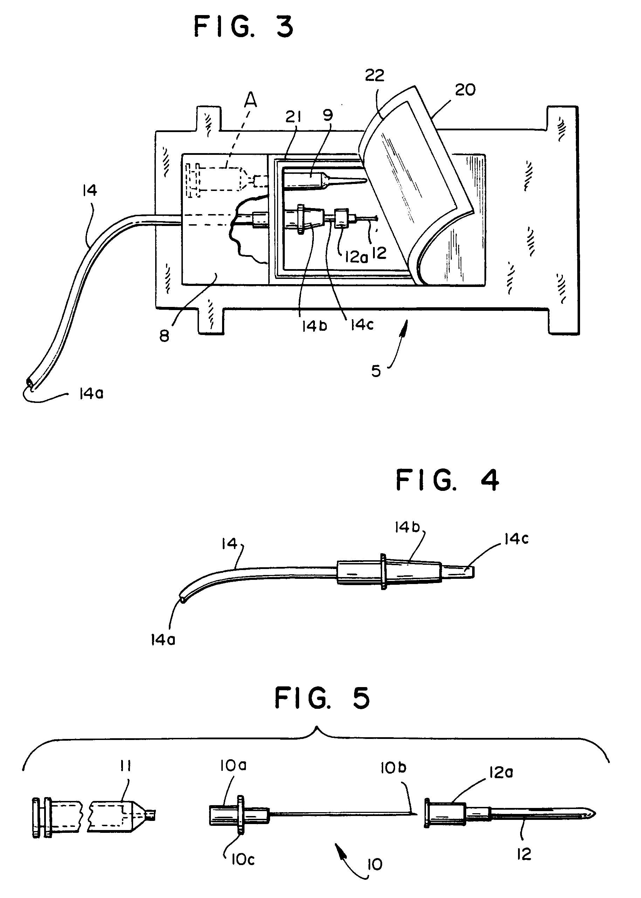

Fig. 1 shows the cassette 1 of the invention within sterile packaging P. Also within packaging P is cover 20 (Fig. 3), which is enclosed within its own sterile packaging (not shown).

After packaging P is opened and discarded, the user removes and discards paper or plastic covers 2 and 3 as described below, which are removably adhesively secured to the open top 4a and open bottom 4b of wall 4. Fig. 2 shows the cassette with covers 2 and 3 removed.

Wall 4 is preferably of rigid plastic and is mounted on and projects from the top surface of the elongated, flexible support 5, which has an outer edge 5a and an inner edge 5b defining an opening 6. The interior surface 4c of wall 4 is located at the inner edge 5b of the support 5 and thus surrounds the opening 6. Support 5 is suitably made of flexible plastic or fabric so as to be easily secured to the skin of a patient. Suitable means is provided, such as adhesive 5c (Fig. 2) on the bottom surface of support 5, for this purpose.

As best seen in Fig. 1, wall 4 has an entrance 7 at one end to permit one to gain access to the interior of wall 4. Secured to wall 4 is a flexible, transparent bag 8, which is made of thin but strong plastic, with the open end 8a hermetically sealed to the cassette 1. Suitably, the open end 8a is sealed to the wall 4 around entrance 7 and to support 5 in front of entrance 7 by means of a suitable adhesive. If the open end 8a is secured at the entrance 7 as shown, the bag 8 can move freely up or down or from side-to-side. If a portion of end 8a is secured to the support 5 in front of entrance 7, the bag 8 can be made taller than shown to provide the desired degree of vertical movement.

Wall 4 includes a needle assembly support 9 projecting into the interior of the wall 4 adjacent entrance 7. Removably housed within needle assembly support 9 is assembly A comprising a hollow needle 10 (Fig. 5) having a hub 10a and a pointed end 10b. Detachably connected to hub 10a is syringe 11 through a conventional friction fit (Fig. 2). Plastic cannula 12 is telescoped over the needle 10 as is conventional.

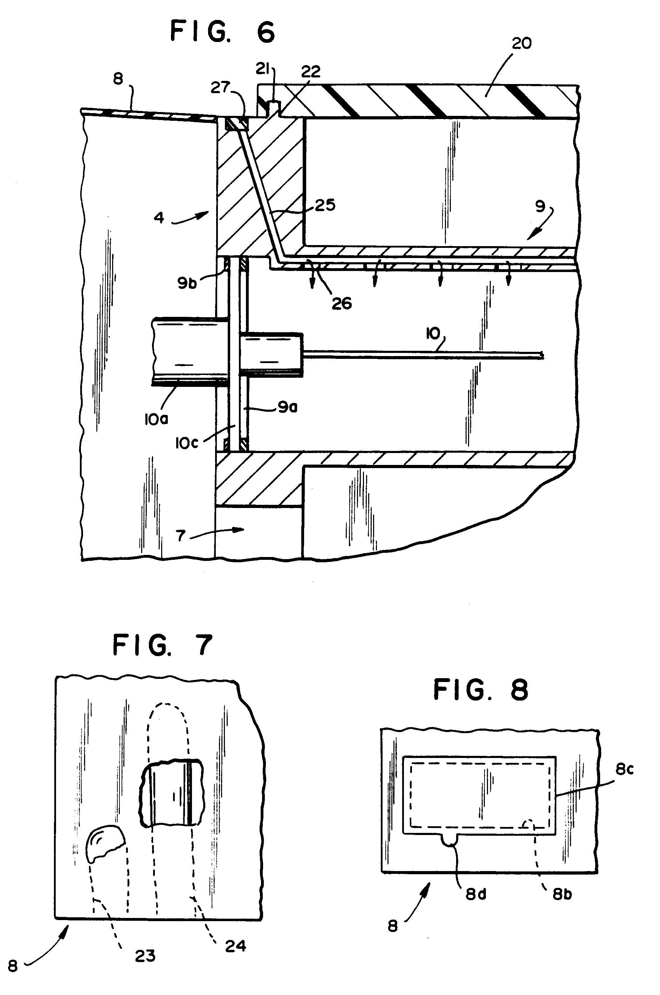

As best seen in Fig. 6, needle 10 is removably held in support 9 with flange 10c between ring 9a and circumferentially spaced members 9b. Fig. 6 shows the needle 10 held by support 9 with the cannula 12 removed. As can be seen, members 9a and 9b cooperate with flange 10c to detachably hold needle 10 in the support 9 whether or not the cannula 12 is carried by the needle 10.

Completing the assembly is tubing 14, which is sealed to bag 8 and which has an end 14a outside the bag 8 and an end 14b inside bag 8. In particular, end 14b is provided with a conventional female fitting 14c, which is designed to be friction fitted within hub 12a (Fig. 3) of cannula 12, as is conventional.

The cassette 1 is used as follows. First, the cassette 1 and cover 20 are removed from packaging P, cover 20 being reserved for later use. Bottom cover 3 is removed and support 5 attached to the skin of the patient. Cover 2 is then removed and the area exposed within the open top 4a is cleansed and then irradiated with ultraviolet light in a manner known per se. This sterilizes the exposed skin within the operating field.

Cover 20 is removed from its sterile packaging (not shown) and is secured to top 4a by suitable means, such as the bead 21 and groove 22 (Fig. 3). It is noted that Fig. 3 shows cover 20 partially removed. This is for clarity only. Once cover 20 is installed, it is intended to remain secured to wall 4 until after cannula 12 is removed from the body in order to maintain the sterility of the operating field at all times.

Needle assembly A is then withdrawn from support 9 by gripping syringe 11 through flexible bag 8. If desired, bag 8 may have inwardly projecting thumb portion 23 and finger portion 24 (Fig. 7). The user will hold the syringe 11 with one hand and will enter a desired blood vessel by means of pointed end 10b of needle 10, which projects beyond plastic cannula 12, as is known. When cannula 12 is inserted, syringe 11 is used to withdraw blood from the vessel to ensure that a blood vessel was indeed entered, as is known.

If the cannula 12 was properly inserted into a blood vessel, syringe 11 and needle 10 are withdrawn, leaving cannula 12 in place. One hand withdraws the syringe 11 and needle 10, while the other holds the cannula 12 in place, if necessary, through bag 8. The used needle 10 is then replaced in support 9, as shown in Fig. 6. To minimize growth of microorganisms on the used needle 10, wall 4 is provided with conduit 25 having exit ports 26. Inlet 27, made of a self-healing membrane, closes conduit 25. A suitable anti-microbial agent can be admitted into the interior of support 9 and into contact with needle 10 by injecting it into conduit 25 through membrane 27.

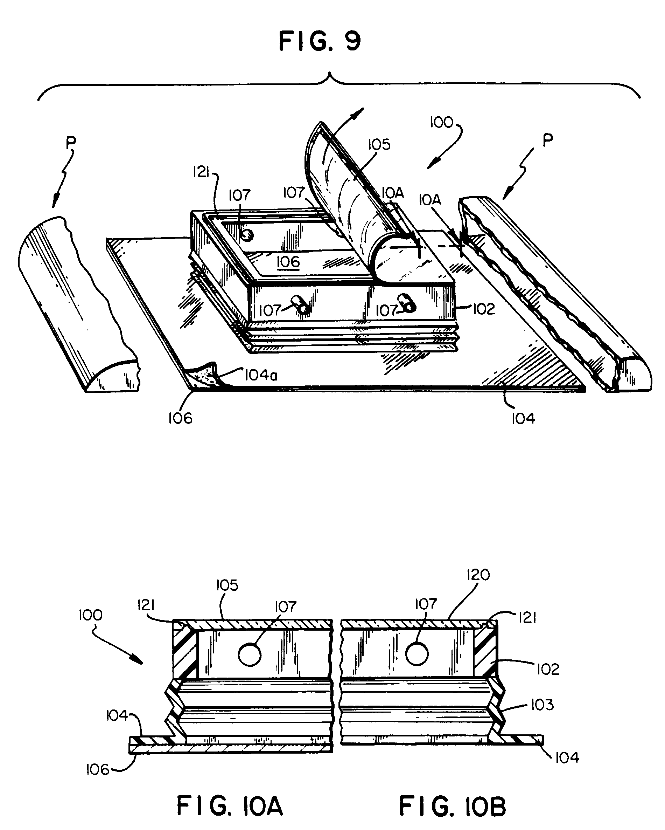

Additional self-healing membranes 28 and gas inlet ports 29 are provided in wall 4 for admitting liquid or gaseous media into the interior of the cassette. For example, sterilized gas under atmospheric pressure can be admitted into the cassette 1 through one port 29 and exhausted from the cassette 1 by the other port 29, thereby contacting the operating field and providing the desired effect of sterilizing the operating field and/or promoting healing. For example, high levels of oxygen are known to promote healing and to prevent growth of anaerobic bacteria. Ozone is known as a sterilizing gas. Hence, oxygen or ozone, are suitable gases for use in this embodiment of the invention. This is explained in detail below in connection with Figs. 9-12.

With cannula 12 in place, tubing 14 is connected to hub 12a by means of fitting 14c. Thereafter, the end 14a of tubing 14 is connected to a reservoir (not shown) of the desired fluid. End 14a is provided with a conventional cap (not shown) that is detachably sealed to end 14a to maintain the sterility of the interior of tubing 14.

While the wall 4 has been shown as rectangular, other shapes are possible, such as oval or circular.

Where the cassette 1 is used for intramuscular administration, the syringe 11 may be used simply as a device for assisting in handling needle 10 and cannula 12 or it may be omitted from the needle assembly A, in which case needle 10 need not be hollow.

Cassette 1 may be of any convenient size to provide opening 6 with dimensions suitable for use on the human body, such as from about 40 to about 60 mm wide to about 40 to about 90 mm long, depending upon the length of needle 10 and the size of the area available for the dermal puncture.

Fig. 8 shows a portion of a flexible bag 8 having an opening or access port 8b closed by lid or cover 8c. Cover 8c is secured to bag 8 by any rapidly detachable means (not shown), such as a bead and groove interlock as in elements 21,22 or by a peelable adhesive, in order to gain rapid access to the operating field via access port 8b in an emergency. Tab 8d facilitates rapid removal of cover 8c.

As seen in Fig. 9, cassette 100 of the invention is stored within sterile packaging P and comprises a rigid tubular wall 102 having an open top end and open bottom end, a flexible bellows-like skirt 103 depending from the bottom end of tubular wall 102 and a flexible flange portion 104 extending away from the skirt 103. Protective sheets 105,106 suitably made of paper or plastic, are removably adhesively secured to the top of wall 102 and across the entire underside of flange portion 104 (Fig. 10A), respectively. In particular, sheet 105 carries a pressure-sensitive adhesive (not shown) for removably adhering to wall 102, whereas sheet 106 removably adheres to the pressure-sensitive adhesive 104a (Fig. 9) on the underside of flange portion 104.

Wall 102 is preferably made of rigid plastic so as to withstand handling during use. Skirt 103 is preferably of flexible rubber or plastic and may be pleated as shown so as to conform to the area of the dermis of the patient where the operating field is to be established. Skirt 103 may be secured to wall 102 by adhesive or by heat welding, as desired. Flange 104 is preferably integral with skirt 103, and is also sufficiently flexible to conform to the dermis and create a gas-tight seal around the operating field. Adhesive 104a may be any suitable pressure-sensitive adhesive, such as used in connection with wound dressings.

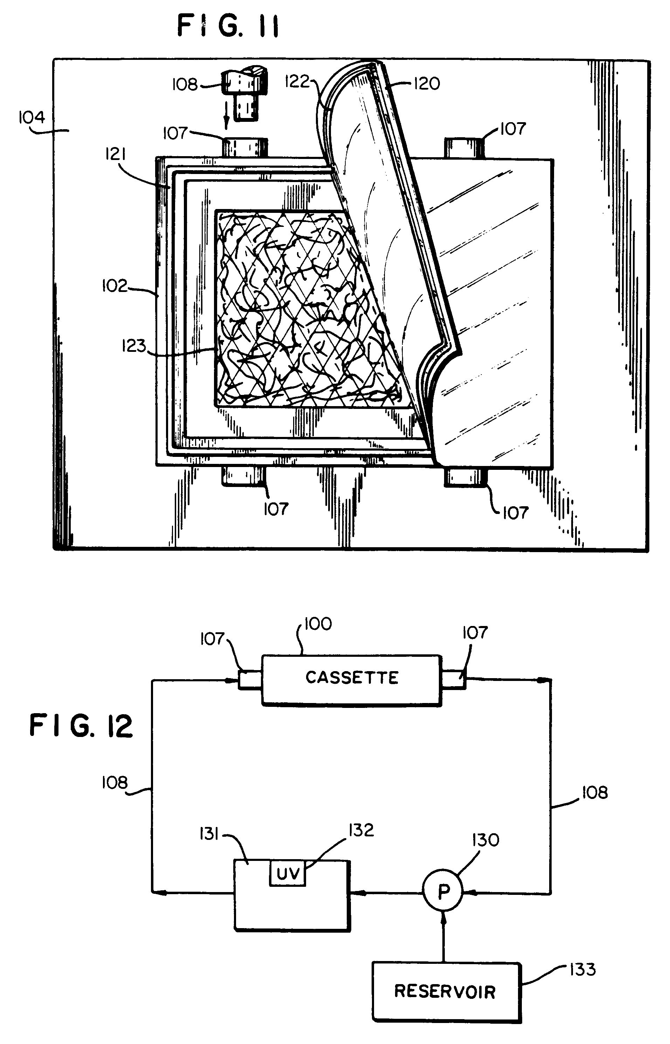

Ports 107 communicate with the interior of cassette 100 and are provided with suitable means (not shown) for attachment to conduits 108 (Fig. 12), which are used to flow a sterilizing gas into and out of the cassette 100 as will be described below. Such attachment means may be suitable fittings, such as used in connecting IV assemblies together as illustrated in Fig. 11.

Separately stored in its own sterile packaging (not shown) is transparent cover 120 (Fig. 11), which will be described hereinafter. Any suitable transparent plastic may be used for cover 120.

After packaging P is opened, the user removes and discards protective covers 105,106 from cassette 100 and secures the cassette 100 to the dermis by means of adhesive 104a on the underside of flange 104. The operating field within wall 102 is cleansed before or after the cassette 100 is affixed to the dermis and is irradiated through the open top of cassette 100 with ultraviolet light in a manner known per se. This sterilizes the exposed skin within wall 102. Alternatively, the operating field may be irradiated with UV light before the cassette 100 is affixed to the patient.

Cover 120 is removed from its sterile packaging (not shown) and is secured to wall 102 by suitable means, such as bead 121 and groove 122 (Fig. 10B). It is noted that Fig. 11 shows cover 12O partially pulled back for clarity only. Once cover 12O is installed, it is intended to remain secured to wall 102 in order to maintain the sterility of the operating field at all times.

With the cassette 100 in place, each conduit 108 (Fig. 12) is attached at one end to a port 107 on either side of cassette 100 and at the other end to pump 130 and chamber 131, respectively. Pump 130 may be any type of medically acceptable pump and is used to pump a sterilizing gas, such as oxygen or ozone, in a closed loop through chamber 131 to cassette 100 and back to chamber 131. Chamber 131 is provided with an ultraviolet lamp 132, such as used with drinking water dispensers, that irradiates the recycled sterilizing gas exhausted from cassette 100 with an effective dose of ultraviolet radiation to sterilize the gas.

Sterilizing gas is thus admitted into the cassette 100 through one port 107 and exhausted from the cassette 100 by the other port 107, thereby contacting the operating field and providing the desired effect of sterilizing the operating field and/or promoting healing. For example, high levels of oxygen are known to promote healing and to prevent growth of anaerobic bacteria. Ozone is known as a sterilizing gas. Hence, oxygen or ozone are suitable gases for use in the present invention.

Sufficient sterilizing gas to start the cycle and to replenish losses is obtained from reservoir 132. Appropriate pressure control equipment (not shown) is preferably used to maintain a pressure slightly above atmospheric, such as about 35.10³ Pa (0.5 psig) to about 35.10³ Pa (5 psig), in order to prevent inflow of airborne organisms into the cassette 100, although it will usually suffice to use a fan or the like as pump 130, whereby the sterilizing gas will be at atmospheric pressure.

Fig. 11 shows a surgical or wound dressing 123 within wall 102 of cassette 100. Suitably, the dressing 123 can be applied to the skin before or after the cassette 100 is secured to the skin, and preferably after the operating field has been irradiated.

When dressing 123 is to be changed, the flow of sterilizing gas is discontinued, after which cover 120 is removed to permit dressing 123 to be removed and replaced, if necessary. The operating field is then irradiated with UV light as described above and a new sterile cover 120 is removed from its sterile packaging (not shown) and secured to cassette 100, whereafter the flow of sterilizing gas is again commenced.

Cassette 100 may be of an convenient size to provide opening 106 with dimensions suitable for use on the human body, such as from about 40 to about 60 mm wide and about 40 to about 90 mm long, depending on the size of the area to be enclosed. The height of wall 102 and bellows 103 may be from about 10 mm to about 30 mm each, or any other convenient size. Preferably, flange 104 extends from about 20 to about 50 mm away from bellows 103. If desired, surgical tape or the like can be placed over flange 104 and onto the patient to provide more secure placement of cassette 100.

Cover 120 is transparent in order to allow viewing of the operating field, and any suitable transparent flexible plastic material may be used as cover 120.

It is contemplated that cassette 100 will be provided in a series of sizes, together with templates that define the area enclosed by wall 102. The templates are used to identify the location and size of the pre-operative area of the skin to be prepped so that it will fit within a selected cassette 100. Each template will be provided within its own sterile packaging. Preferably, the templates are congruent to the opening in skirt 103, but they can be a smaller size, if desired.

Of course, cassette 1 of Figs. 1-8 may be used in the above-described sterilization method instead of cassette 100, in which case the sterilizing gas may be flowed into contact with the operating field therein by connecting conduits 108 of the system of Fig. 12 hereof to the ports 29 of the cassette 1 after the cassette 1 and cover 2 are installed on the patient.

## Claims

1. A cassette assembly having :
A. a first sterile packaging enclosing
a sterile cassette (1) comprising an elongated flexible support (5) having top and bottom surfaces, an outer edge (5a) defining the boundary of said support, an inner edge (5b) defining an opening (6) in said support, and means (5c) for attaching said bottom surface to the skin of a patient, and
elongated wall means (4) projecting from said top surface of said support (5) and extending around said inner edge (5b), said wall means (4) having an inner surface (4c) enclosing said opening (6), said wall means (4) further having an open top (4a), an open bottom (4b), and an entrance (7) at one end ;
B. a sterile transparent cover means (20) packaged within a second sterile packaging,
characterized by the first sterile packaging further enclosing :
a flexible transparent bag (8) having opposed open and closed ends (8a) connected at its open end to said wall means (4) and closing said entrance (7), said entrance (7) providing free communication between the interior of said bag (8) and the interior of said wall means (4) ;
tubing (14) sealed to and passing through said bag (8) with one end (14b) inside and the other end (14a) outside said bag (8) ;
a needle assembly support (9) carried by said wall means (4) adjacent said entrance (7) and projecting into the interior of said wall means (4) ;
a needle assembly comprising a needle (10) having a hub (10a) at one end and a point (10b) at the other, and a cannula (12) removably telescoped over said needle (10) with said pointed end (10b) exposed, said needle assembly being supported inside said needle assembly support (9) with said hub (10a) projecting away from said needle assembly support (9) and into said bag (8) ;
said needle assembly support (9) including means for removably holding said needle with or without said cannula (12) telescopically mounted thereon ; and
cover members (2,3) for removably adhesively closing said open top (4a) and open bottom (4b) of said wall means (4).

2. The cassette assembly according to claim 1, characterized in that said needle (10) is hollow and a syringe (11) is secured to said hub (10a) in fluid communication with said hollow needle (10).

3. The cassette according to claim 2, characterized in that said wall means (4) includes conduit means (25) for introducing a liquid into said needle assembly support (9) and into contact with a needle (10) supported thereby.

4. The cassette assembly according to one of claims 1 to 3, characterized in that said wall means (4) includes ports (29) for admitting a fluid into the interior of said wall means (4), and closures for closing said ports.

5. The cassette assembly according to one of claims 1 to 4, characterized in that said bag (8) has finger shaped portions (23,24) extending inwardly therein.

6. The cassette assembly according to any one of claims 1 to 5, characterized in that said closed end of said bag (8) has a rapid access port (8b) therein closed by a lid (8c) detachably secured to said bag for rapid removal therefrom.

## Patentansprüche

1. Kassettenaufbau mit:
A: einer ersten sterilen Verpackung, die einschließt:
eine sterile Kassette (1), die eine längliche, flexible Halterung (5), die eine obere und untere Fläche, eine Außenkante (5a), die die Grenze der Halterung festlegt, eine Innenkante (5b), die eine Öffnung (6) in der Halterung festlegt, sowie eine Einrichtung (5c) besitzt, um die untere Fläche auf der Haut eines Patienten zu befestigen, sowie
ein längliches Wandelement (4) enthält, das von der oberen Fläche der Halterung (5) vorspringt und um die Innenkante (5b) verläuft, wobei das Wandelement (4) eine Innenfläche (4c) besitzt, die die Öffnung (6) umschließt, wobei das Wandelement (4) weiters eine offene Oberseite (4a), einen offenen Boden (4b) sowie an einem Ende einen Zugang (7) besitzt;
B: einem sterilen, durchsichtigen Abdeckelement (20), das in einer zweiten sterilen Verpackung verpackt ist,
dadurch gekennzeichnet, daß die erste sterile Verpackung weiters einschließt:
eine flexible, durchsichtige Tasche (8), die gegenüberliegend ein offenes und ein geschlossenes Ende (8a) besitzt, wobei sie an ihrem offenen Ende mit dem Wandelement (4) verbunden ist und den Zugang (7) verschließt, wobei der Zugang (7) die freie Verbindung zwischen dem Inneren der Tasche (8) und dem Inneren des Wandelements (4) liefert;
eine Rohrleitung (14), die mit der Tasche (8) abgedichtet ist und diese durchläuft, wobei ein Ende (14b) innerhalb und das andere Ende (14a) außerhalb der Tasche (8) liegt;
einen Nadelhalterungsaufbau (9), der vom Wandelement (4) neben dem Zugang (7) gehalten wird und in das Innere des Wandelements (4) vorspringt;
einen Nadelaufbau, der eine Nadel (10), die an einem Ende eine Nabe (10a) und am anderen Ende eine Spitze (10b) besitzt, sowie eine Kanüle (12) enthält, die abziehbar über die Nadel (10) geschoben wird, wobei das spitze Ende (10b) freiliegt, wobei der Nadelaufbau innerhalb der Nadelaufbauhalterung (9) mit der Nabe (10a) gehalten wird, die von der Nadelaufbauhalterung (9) in die Tasche (8) vorspringt;
wobei die Nadelaufbauhalterung (9) eine Einrichtung aufweist, um die Nadel mit oder ohne Kanüle (12) abnehmbar zu halten, die teleskopartig daran befestigt ist; sowie
Deckelelemente (2, 3), um die offene Oberseite (4a) und den offenen Boden (4b) des Wandelements (4) abnehmbar anhaftend zu verschließen.

2. Kassettenaufbau gemäß Anspruch 1, dadurch gekennzeichnet, daß die Nadel (10) hohl ist, wobei eine Spritze (11) an der Nabe (10a) befestigt ist, die mit der Hohlnadel (10) fluidmäßig in Verbindung steht.

3. Kassette gemäß Anspruch 2, dadurch gekennzeichnet, daß das Wandelement (4) eine Kanaleinrichtung (25) aufweist, um eine Flüssigkeit in die Nadelaufbauhalterung (9) sowie in Berührung mit einer davon gehaltenen Nadel (10) einzuleiten.

4. Kassettenaufbau gemäß irgendeinem der Ansprüche 1 bis 3, dadurch gekennzeichnet, daß das Wandelement (4) Öffnungen (29), um ein Fluid in das Innere des Wandelements (4) einzuleiten, sowie Verschlüsse aufweist, um diese Öffnungen zu verschließen.

5. Kassettenaufbau gemäß irgendeinem der Ansprüche 1 bis 4, dadurch gekennzeichnet, daß die Tasche (8) fingerförmige Teile (23, 24) besitzt, die in ihrem Inneren verlaufen.

6. Kassettenaufbau gemäß irgendeinem der Ansprüche 1 bis 5, dadurch gekennzeichnet, daß das geschlossene Ende der Tasche (8) eine Schnellzugangsöffnung (8b) darin aufweist, die mit einem Deckel (8c) verschlossen ist, der an der Tasche abnehmbar so befestigt ist, daß er rasch davon abgenommen werden kann.

## Revendications

1. Ensemble à cassette ayant :
A. un premier paquet stérile renfermant
une cassette stérile (1) comprenant un support souple allongé (5) ayant une face supérieure et une face inférieure, un bord extérieur (5a) formant la limite de ce support, un bord intérieur (5b) délimitant une ouverture (6) faite dans ce support, et un moyen (5c) de fixation de la face inférieure à la peau d'un malade, et
des moyens de paroi allongés (4) saillant de la face supérieure du support (5) et s'étendant autour du bord intérieur (5b), ces moyens de paroi (4) ayant une face intérieure (4c) enfermant l'ouverture (6), ces moyens de paroi (4) ayant en outre un dessus ouvert (4a), un dessous ouvert (4b) et une entrée (7) à une extrémité,
B. un moyen de couverture transparent stérile (20) empaqueté dans un second paquet stérile,
caractérisé par le fait que le premier paquet stérile renferme en outre :
un sac souple transparent (8) ayant une extrémité ouverte et une extrémité opposée fermée (8a), joint à son extrémité ouverte aux moyens de paroi (4) et fermant l'entrée (7), l'entrée (7) assurant une libre communication entre l'intérieur du sac (8) et l'intérieur des moyens de paroi (4),
un tube (14) soudé au sac (8) et le traversant avec une extrémité (14b) à l'intérieur et l'autre extrémité (14a) à l'extérieur de celui-ci,
un support d'ensemble à aiguille (9) porté par les moyens de paroi (4) près de l'entrée (7) et saillant à l'intérieur des moyens de paroi (4),
un ensemble à aiguille comprenant une aiguille (10) ayant un moyeu (10a) à une extrémité et une pointe (10b) à l'autre extrémité, et une canule (12) placée de manière amovible sur l'aiguille (10) avec l'extrémité pointue (10a) en dehors, cet ensemble à aiguille étant supporté à l'intérieur du support d'ensemble à aiguille (9) avec le moyeu (10a) saillant de celui-ci et entrant dans le sac (8),
le support d'ensemble à aiguille (9) comportant un moyen de fixation amovible de l'aiguille avec ou sans la canule (12) montée dessus, et
des éléments de couverture (2, 3) destinés à fermer par adhérence le dessus ouvert (4a) et le dessous ouvert (4b) des moyens de paroi (4).

2. Ensemble à cassette selon la revendication 1, caractérisé par le fait que l'aiguille (10) est creuse et une seringue (11) est fixée au moyeu (10a), en communication avec l'aiguille creuse (10).

3. Ensemble à cassette selon la revendication 2, caractérisé par le fait que les moyens de paroi (4) comportent un conduit (25) pour l'introduction d'un liquide dans le support d'ensemble à aiguille (9) et la mise en contact de ce liquide avec une aiguille (10) supportée par ce support (9).

4. Ensemble à cassette selon l'une des revendications 1 à 3, caractérisé par le fait que les moyens de paroi (4) comportent des orifices (29) pour l'admission d'un fluide à l'intérieur des moyens de paroi (4), et des moyens de fermeture de ces orifices.

5. Ensemble à cassette selon l'une des revendications 1 à 4, caractérisé par le fait que le sac (8) a des parties en forme de doigt (23, 24) qui s'étendent vers l'intérieur dans celui-ci.

6. Ensemble à cassette selon l'une des revendications 1 à 5, caractérisé par le fait que l'extrémité fermée du sac (8) contient un orifice d'accès rapide (8b) fermé par un couvercle (8c) fixé au sac de façon à pouvoir en être enlevé rapidement.
